# EUROPEAN PATENT APPLICATION

(11) **EP 4 556 057 A1**
(43) Date of publication of application: **21.05.2025**
(21) Application number: 23383170.0
(22) Date of filing: 16.11.2023
(51) Int. Cl.: A61N 1/36, A61N 1/04

(54) **DEVICE AND METHOD FOR REHABILITATION OF UPPER LIMB FUNCTION BY FUNCTIONAL ELECTRICAL STIMULATION**

(71) Applicant: FESIA Technology, S.L., 20009 Donostia (Gipuzkoa) (ES)
(72) Inventor: RODRÍGUEZ DE PABLO, Cristina, 20009 San Sebastian (Gipuzkoa) (ES); DORRONSORO ESNAL, Iñigo, 20009 San Sebastian (Gipuzkoa) (ES); ZABALETA REKONDO, Haritz, 20009 San Sebastian (Gipuzkoa) (ES)
(74) Representative: Balder IP Law, S.L.

(57) **Abstract**

A device (1) and method for functional electrical stimulation intended to be positioned on an upper limb of a user for stimulation of the user's upper limb, comprising an electrode (2) configured to be disposed on an upper limb of the user, the electrode (2) having a plurality of electrode pads (3) adapted to generate stimulating electrical signals on the upper limb of the user, and a stimulator (4) comprising an embedded sensor (5) configured to detect a movement of the stimulator (4), and control means (6) configured to process the sensor measurements and determine an intention of the user to perform an action with his/her upper limb on which the electrode (2) is disposed, and to control the activation and/or deactivation of the electrode pads (3) based on the sensor measurements.

## Description

### TECHNICAL FIELD

The present invention relates to the field of functional electrical stimulation and, in particular, to apparatuses, systems and methods for rehabilitation of paralyzed or impaired body parts, such as hands and/or arms, by electrostimulation of peripheral nerves.

### STATE OF THE ART

Neurological injuries or disorders, such as stroke, spinal cord injury or cerebral palsy, among others, usually cause complete or partial paralysis or severe weakness in certain body parts. This often results in impaired or lost function in one or more limbs. It is well-known that people affected by these traumas can rehabilitate significantly by executing familiar and/or functional tasks. Assistance for performing this rehabilitation is usually required, since after neurological injuries the patient is usually diminished in his/her capacity to control or to strength the afflicted limb or limbs. Wearable devices have been proven to be of effective assistance in such rehabilitation tasks.

There exist electro-mechanical wearable rehabilitation devices. For example, EP3199136B1 discloses a portable device for hand rehabilitation designed to be grasped by a hand to be trained. This device enables independent flexion/extension movement for the thumb, index fingers and three remaining fingers. In turn, US9398994B2 discloses an orthotic system for limb rehabilitation including a brace having two pivotably sections, an electrically powered actuator assembly and an electroencephalographic (EEG) sensor. The actuator assembly applies a force that moves the brace sections in flexion/extension direction. The applied force is based on signals from the EEG sensor.

Functional electrical stimulation (FES) is a technique that uses low energy electrical pulses to artificially generate muscle contractions to produce body segment movements and motor functions such as grasping or walking. There exist wearable rehabilitation devices for external activation of paralyzed or impaired body parts by stimulation of peripheral nerves based on FES. For example, EP2519312B1 discloses a sleeve designed to be worn by a user having a stimulation layer having multi-pad electrodes designed to be in contact with the user's skin, and an activation layer opposite the stimulation layer and overlapping it, composed of pressure-driven sensors for activating/deactivating corresponding pads of the stimulation layer.

A challenge of FES devices for the upper-limb is to determine the proper activation means so that they can be integrated in the daily activities as currently do FES devices for the lower-limb. Current FES devices require the use of external sensors to control the application of stimulating signals.

For example, the MindMaze group provides (https://www.intento.ch/movement-therapy/, last access of October 11, 2023) a FES device having electrodes to be placed on specific areas of the patient's arm to stimulate muscular contractions and induce the desired movements. The patient controls the stimulation by an external device activated with the other hand. Bioness (https://www.bioness.com/, last access of November 08, 2023) also offers the H200^{®} wireless system, which uses FES to activate the nerves that control the muscles in the hand and forearm and is controlled from a hand-held control unit.

A drawback with the former proposals is that they require the impaired person to hold a control device in his/her hand different from the hand or arm being rehabilitated. However, depending on the condition of the user, this requirement can become an insurmountable obstacle for the patient.

Devices have been developed, which do not rely on a hand-held device portable with the hand not being under rehabilitation. For example, EP3650077A1 discloses a FES device for limb rehabilitation which includes a garment mountable on a user's body part. The garment holds an electrode and a socket in turn housing a stimulator. The stimulator adapts the stimulating electrical signals to be provided to the electrode from sensor signals provided by an external sensor that measures the movement resultant from the stimulation; however, the trigger for that stimulation is sent from an external control means as a tablet or smartphone.

In turn, EP1555968B1 discloses a detector disposed on the user's head to control signals applicable to electrodes to stimulate muscles to open and/or close thumbs and fingers of the hand. The detector detects mechanical vibrations elicited by sudden contact of upper and lower teeth and these vibrations are used to control the operation of the electrodes.

Despite the efforts to accommodate current devices to the condition of the user, there is still a need to reduce complexity in terms of the equipment the patient must wear or carry, with the aim to simplify as much as possible the rehabilitation process and to enable the patient to perform the daily activities in total autonomy.

### DESCRIPTION OF THE INVENTION

The drawbacks of conventional FES devices are addressed by the present invention, which provides a functional electrical stimulation device which is very easy to use in an autonomous way, even for a person having suffered a neurological injury. The device detects a user's intention to perform an action with the upper limb and stimulates the muscles required to do so. The upper limb may include the hand, forearm, elbow, arm and/or shoulder.

The device improves upper limb function and active range of motion in adults and pediatric individuals with injury or pediatric-related motor dysfunction related to injuries or diseases affecting the physiological functioning of the upper limb (e.g., stroke, paralysis of the upper limb, etc.).

Particularly, the device electrically stimulates the muscles and nerves of the affected upper limb to provide joint flexion and extension. It also facilitates muscle re-education, it prevents or retards atrophy of said muscles, it helps to maintain or increase joint range of motion and it increases local blood flow.

The device for functional electrical stimulation of the present disclosure is intended to be positioned on an upper limb of a user for stimulation of the user's upper limb, such as its hand and/or arm.

In a first aspect of the invention, a device for functional electrical stimulation intended to be positioned on an upper limb of a user for stimulation of the user's upper limb, is provided. The device comprising:
- an electrode configured to be disposed on an upper limb of the user, the electrode having a plurality of electrode pads adapted to generate stimulating electrical signals on the upper limb of the user, and
- a stimulator comprising:
   - an embedded sensor configured to, in use of the device, continuously measure movement of the stimulator, and to emit sensor measurements indicative thereof, and
   - control means connected to the embedded sensor and configured to process the sensor measurements and from said measurements to determine an intention of the user to perform an action with his/her upper limb on which the electrode is disposed, and to control the activation and/or deactivation of at least one of the plurality of electrode pads based on the sensor measurements.

As previously explained, the embedded sensor continuously measures the presence or lack of movement of the stimulator. In the context of the present invention, the term "continuously" means that the measurements captured by the sensor are captured at a frequency above a certain value, such as above 20 Hz, or above 60 Hz, or above 120 Hz. This means that the sensor obtains measurements, and it sends them to the control means in a high enough frequency, e.g., 125 Hz, to permit fine movement detection.

As a skilled person in the art knows, the stimulating electrical signals generated on the upper limb of the user by the electrode are stimulation patterns, such as pulse patterns with certain characteristics or parameters, such as frequency, pulse width, amplitude, compensation, time duration, etc., which cause the contraction or relaxations of the muscles.

In affected users, when they want to perform an action, the stimulus generated by the brain does not reach the muscle, which does not move as it should. Thanks to the device of the invention the muscle contracts by the stimulation. Thus, the brain receives information about the movement and strives to control it, creating new connections as another area of the brain controls the movement, recovering autonomy.

Both the sensor and the control means are embedded in the stimulator. Communication between the embedded sensor and the control means is preferably via a wired connection. The device also has the advantage, compared to the devices of the state of the art, that it eliminates external elements, allowing bimanual tasks (cannot be done with a remote control in the other hand), avoiding the loss of said external elements, and also for aesthetic reasons.

In embodiments of the invention, the stimulator is intended to be attached to the user's arm.

In embodiments of the invention, the movement of the stimulator associated with an intention of the user to perform an action with his/her upper limb is a sharp or abrupt movement of the stimulator followed and preceded by a rest period. A rest period is a period where the control means of the stimulator have not detected any movement or the movement detected is below a predefined threshold. A sharp or abrupt movement is therefore a short movement preceded and followed by a rest period. For example, in embodiments of the invention, a short movement could last a couple of milliseconds and the rest period a couple of seconds.

That type of movement is key to the use of the device in the daily life of a user. The device must be easy enough to be used so that users are able to perform the movement freely by themselves, but at the same time it must be a movement that is only performed intentionally to allow it to be differentiated from normal upper limb use and to avoid false positives in the detection of movement intention.

This type of movement can be generated at least in three ways:
- With the movement of the affected arm itself, and without external elements. This movement has two main advantages. The first one, as an assistive device, as it allows the performance of bimanual tasks and the use of the arm in activities of daily living. The second one, as a rehabilitative device, as it promotes the use of the affected arm, which is key to its rehabilitation.
- With the unaffected arm, in which the stimulator is not placed. It has the advantage that the patient always has the option of using the unaffected arm when for example fatigue prevents him/her from using the affected arm.
- By another person, such as a therapist placed next to the user. It presents the advantage that the device becomes a rehabilitation tool that allows to integrate the device in therapy works.

In embodiments of the invention the movement of the stimulator is generated by at least one of:
- impaired arm flexion, extension, abduction, adduction, medial rotation, lateral rotation, horizontal flexion/adduction, flexion or extension, both with and without hand in a fixed position,
- impaired forearm flexion, extension, pronation, semipronation, supination or prosupinantion, both with and without hand in a fixed position,
- tapping on the stimulator with the unaffected arm or by a third person.

These kinds of movements (in particular, those in the first and second lists above) encourage the use of the affected arm to trigger stimulation and hand opening/closing. Specifically, forearm prosupinantion, abduction with hand in a fixed position and adduction with hand in a fixed position are movements that can be performed while grasping and carrying objects. These movements require stricter rest periods before and after the movement, to avoid false detections and accidentally drop the objects, for example. All the described movements, but particularly the ones that can be performed while grasping objects, enable activities of daily living.

In embodiments of the invention, the embedded sensor is an IMU (inertial measurement unit). This sensor is very precise and obtains very accurate measurements, allowing optimum calibration of the device to ensure detection of movement without giving false positives. In embodiments of the invention the IMU is a three axis IMU. In other embodiments of the invention the IMU is a six axis IMU. In embodiments of the invention the embedded sensor is a 6-axis IMU, comprising a 3-axis gyroscope and a 3-axis accelerometer sensor. In embodiments of the invention, the embedded sensor is an EMG sensor (electromyography), which records the electrical activity generated by the user's muscles when trying to perform a movement with the upper limb even if they are not able to complete it; therefore, providing information about the movement intention of the user. In embodiments of the invention, the embedded sensor can comprise both, an IMU and an EMG.

In embodiments of the invention, the measurements that the embedded sensor sends to the control means include one or more of the positions of the three axis of the gyroscope and the acceleration measured by the accelerometer. In embodiments of the invention, after the embedded sensor sends its measurements to the control means, the last obtains the modulus of the three axes of the gyroscopes and the modulus of the three axes of the accelerometer. In embodiments of the invention the control means calculate one or more of the Quaternion, Euler angles and rotation vector.

In a second aspect of the invention, a method for rehabilitation of upper limb function by functional electrical stimulation is provided. The method comprising the steps of:
- providing a stimulator having an embedded sensor,
- measuring by the sensor movement of the stimulator and emitting sensor measurements indicative thereof,
- providing the sensor measurements to control means,
- determining, with the control means, an intention of the user to perform an action with his/her impaired upper limb, wherein said intention to perform the action is determined based on the sensor measurements,
- controlling, with the control means, the activation and/or deactivation of at least one of a plurality of electrode pads comprised in an electrode intended to be positioned on an upper limb of a user.

As with the device previously described, the method of the invention allows a user to perform an upper limb action through electrical stimulation of the upper limb muscles in a simple and comfortable way.

In embodiments of the invention the stimulator having the embedded sensor is attached to a user's arm.

As with the device of the invention, in an embodiment of the invention the movement is generated in one of the following ways:
- impaired arm flexion, extension, abduction, adduction, medial rotation, lateral rotation, horizontal flexion/adduction, flexion or extension, with or without fixed hand or
- impaired forearm flexion, extension, pronation, semipronation, supination or prosupinantion, with or without fixed hand,
- tapping on the stimulator.

In an embodiment of the invention, the movement associated with an intention of the user to perform an action with his/her hand or arm (upper limb) is detected when the control means detect, in the sensor measurements, a value above a predefined threshold preceded by a first rest period and followed by a second rest period.

In embodiments of the invention, an additional period, referred to as waiting period, is defined.

This waiting period is aimed at turning the device to an active mode. For example, the waiting period can follow the second rest period, or the waiting period can precede the first rest period. This waiting mode is very important to avoid false positives, for example if the user has poor control over his/her impaired limb, or, following a rest period, the user can still experience some residual movements after having performed an action. It also helps the patient's rehabilitation by requiring finer control of the arm or hand. During this waiting mode the control means remain inactive. Inactive meaning that the control means do not send any signal to the electrode to activate or deactivate the electrode pads.

The sensor may measure different parameters. In embodiments of the invention, the measurements that the embedded sensor measures and sends to the control means include one or more of the positions of the three axis of the gyroscopes and the acceleration measured by the accelerometers. In embodiments of the invention, after the embedded sensor sends its measurements to the control means, the control means obtains the modulus of the three axes of the gyroscope and the modulus of the three axes of the accelerometer. In embodiments of the invention the control means calculate one or more of the Quaternion, Euler angles and rotation vector.

In an embodiment of the invention, the control means determine that there is an intention to perform a movement if the following conditions are met:
- during a first rest period the sensor measurements are below a first value of the measured parameter,
- during a movement period following the first rest period, the sensor measurements are above a second value of the measured parameter, which is at least twice the first value; in embodiments of the invention, the second value can be at least 3 times the first value, such as at least 5 times, or at least 10 times, or at least 15 times the first value, depending on the way the movement is generated and the characteristics of the user,
- during a second rest period following the movement period the sensor measurements are below a value of the measured parameter, for example the first value.

In preferred embodiments of the invention, the control means determine that there is an intention to perform a movement if all the conditions are met.

An additional moderated rest period can be used between the movement period and the second rest period.

For example, when the sensor is an IMU including a gyroscope, the first value is between 0 and 12 dps (degrees per second), preferably between 0 and 6 dps and more preferably between 0 and 3 dps.

For example, when the sensor is an IMU including a gyroscope, the second value is above 15dps, provided that the second value is at least twice the first value.

In embodiments of the invention, when the sensor is an accelerometer, the control module identifies an intention to perform an action if the module of the acceleration changes, which may be directly related to hit or fast speed change of the stimulator.

For example, the first rest period is between 100 and 250ms, the movement period is between 250 and 1000ms and the second rest period is between 150 and 750ms.

The exemplary values described above ensure an optimum operation of the device.

Additional advantages and features of the invention will become apparent from the detail description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows a general view of a device according to embodiments of the invention, placed on the arm of a user.
Figure 2 shows a view of different elements of a device according to embodiments of the invention.
Figure 3 shows a flow diagram of a device according to embodiments of the invention, in use, where the user grabs and releases and object.
Figure 4 shows different movements that can trigger the activation and/or deactivation of electrode pads of the device.
Figures 5A-5C show the best movements to trigger the activation and/or deactivation of electrode pads of the device when the hand must be in a fixed position.
Figure 6 shows a user triggering the device with its other hand.
Figure 7A shows a graph of a signal representing movement which will be detected by the device. The signal presents a sudden variation preceded and followed by a clear rest period.
Figure 7B shows a graph of a signal representing movement which will not generate the trigger.
Figure 8 shows a schematic representation of the electronics of a stimulator comprised in a device according to embodiments of the invention.
Figure 9 shows a flow diagram of the method according to embodiments of the invention.

### DESCRIPTION OF A WAY OF CARRYING OUT THE INVENTION

The following description is not to be taken in a limiting sense but is given solely for the purpose of describing the broad principles of the invention. Next embodiments of the invention will be described by way of example, with reference to the above-mentioned drawings, showing apparatuses and results according to the invention.

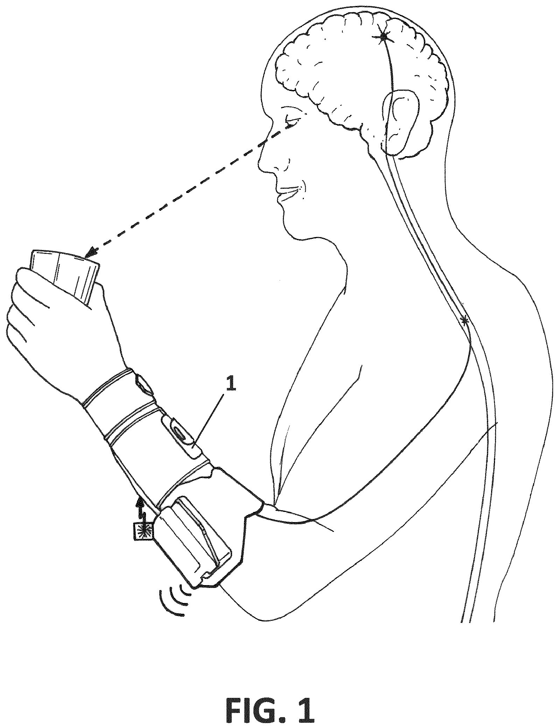

Figure 1 shows a general view of the device 1 for rehabilitation of upper limb function by functional electrical stimulation, according to an embodiment of the present invention. As shown in the figure, the device 1 is placed on an impaired forearm of the user. In this case, the user has performed a movement to indicate that he wants to perform and action, and the device 1 has activated his muscles, so he has been able to grab a glass.

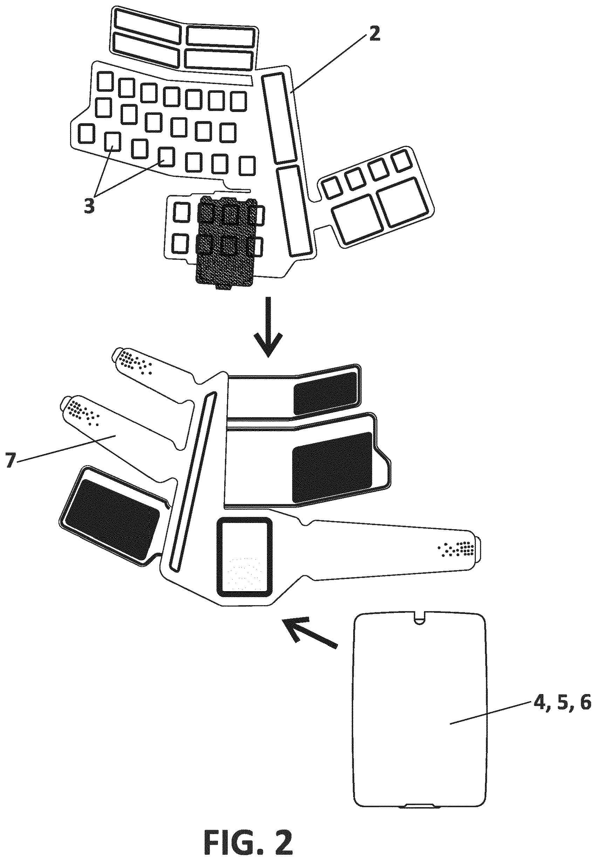

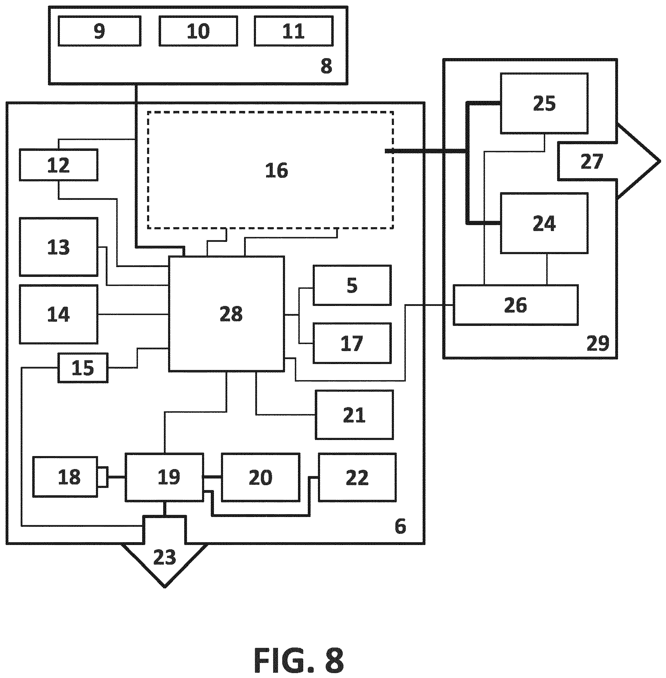

As presented in figure 2, which shows a view of the different elements of an exemplary device 1, it comprises an electrode 2 that is attached to on an arm of the user. The electrode 2 comprises a plurality of electrode pads 3 adapted to generate stimulating electrical signals on the arm of the user. The device 1 further comprises a stimulator 4 which comprises an embedded sensor 5. The embedded sensor 5 is configured to continuously measure a movement of the stimulator 4, and to emit sensor measurements indicative thereof. The device 1 also comprises control means 6, also embedded in the stimulator 4, presented in an exemplary embodiment of the electronics shown in figure 8, connected to the embedded sensor 5 and configured to process the sensor measurements and from said measurements to determine an intention of the user to perform an action with his/her hand or arm on which the electrode 2 is disposed, as shown in figure 1. The intention to perform the action is determined based on the sensor measurements. The control means 6 are also configured to control the activation and/or deactivation of at least one of the plurality of electrode pads 3 based on the sensor 5 measurements.

The electrode pads 3 can be activated independently or in combination, allowing it to be adapted to the anatomy of different users. Also, intensity can be controlled. The generated pulse can be symmetric biphasic or compensated biphasic, for example with a 0-60mA intensity, 150us - 300us width and 1 - 40Hz frequency.

As illustrated in figure 2, the device 1 also comprises a textile support layer 7 with a first face, where the electrode pads 2 are placed, intended to contact the arm or forearm of the user, and a second face, opposite the first face, where the stimulator 4, embedded sensor 5 and control means 6 are placed.

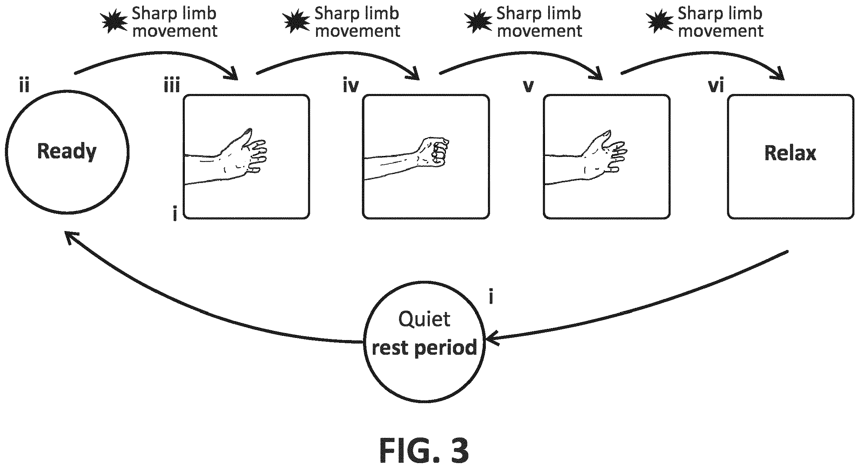

The movement of the stimulator 4 that causes the control means 6 to determine that the user wants to perform an action is a sharp or abrupt movement of the stimulator 4 followed and preceded by a rest period. As an example, figure 3 shows a flow diagram of the device 1 being used, where the user grabs and releases and object. In particular, during i) a quiet rest period occurs, when no movement is detected. During ii) the device 1 is ready to be activated, which happens when iii) the user makes a sharp limb movement, which is registered in the sensor measurements 5, which are send to the control means 6, that determines an intention to perform a movement and controls the electrode 2 in order to produce an electric signal, which makes the user open its hand. Following these actions and after a rest period, the user makes another sharp movement iv), being the previously described steps repeated again and, in this case, the user closes its hand after the electric stimulation. Finally, after another sharp movement v) the hand is opened again and after a final sharp limb movement vi) the muscles relaxes and the device 1 enters into a waiting mode.

Moreover, an additional period, referred to as waiting period, can be defined. This waiting period is aimed at turning the device 1 to an active mode. The waiting period can follow the second rest period, or it can precede the first rest period. In the case of the flow diagram of figure 3 the device 1 enters a waiting mode after the second rest period ends and the specific application example described in figure 3 has reached the end of the flow. The waiting period could be a period of 2 seconds. After the user performs the actions with his/her hand, they can still experience some residual movements that the control means 6 could also identify as an intention to perform an action. In this case, another cycle would start. However, thanks to the waiting mode, the device 1 makes sure that the first cycle is completed, remaining in the waiting mode, and the device 1 does not enter another cycle immediately. In this way, even if the control means 6 identifies an intention to perform an action, if the device 1 is in the waiting mode, the control means 6 does not send any signal to the electrode 2. This waiting mode also helps the patient's rehabilitation by requiring finer control of the arm, as some patients find it challenging to hold their arm still for a two-second waiting period, for example. Nevertheless, when training, this can always be alternatively solved resting the upper limb over a table, for example.

These steps are only an example of the actions that can be performed when the electrodes 2 are activated by the control means 6, when the device identifies a movement as an intention to perform an action. These actions depend on the location and number of electrode pads 3 that are activated.

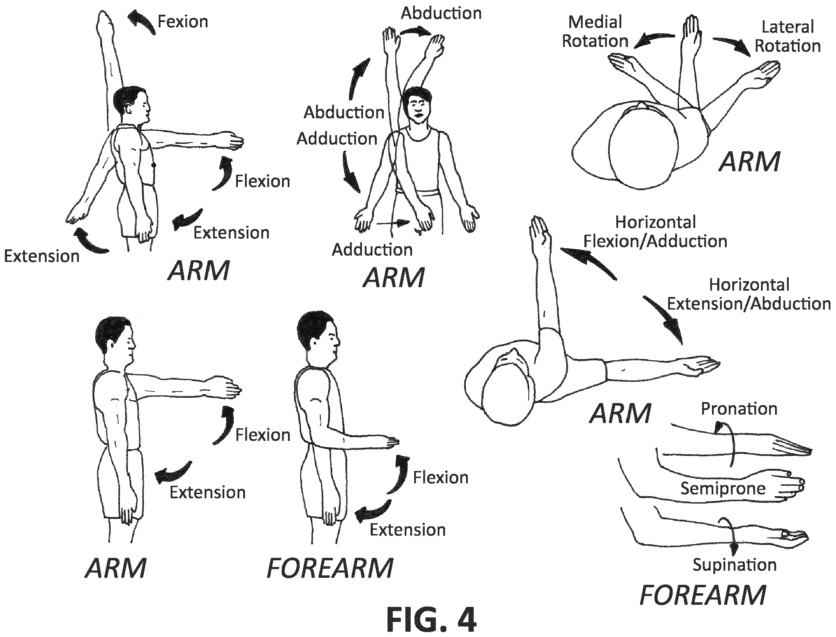

Examples of the types of movement which can make the control means 6 detect an intention to perform an action are depicted in figures 4, 5A-5C and 6. Figure 4 shows several movements, originated in the arm or in the forearm. It is important to note that this is the arm or forearm affected with a mobility problem, which is also the one in which the device 1 of the invention is placed.

The arm movements are depicted on the upper portion of figure 4 and on its lower left corner and are, from left to right and from top to bottom flexion, extension, abduction, adduction, medial rotate, lateral rotation, horizontal flexion/adduction, flexion or extension.

The forearm movements are depicted on the lower portion of figure 4 and are, from left to right forearm flexion, extension, pronation, semipronation, supination or prosupinantion.

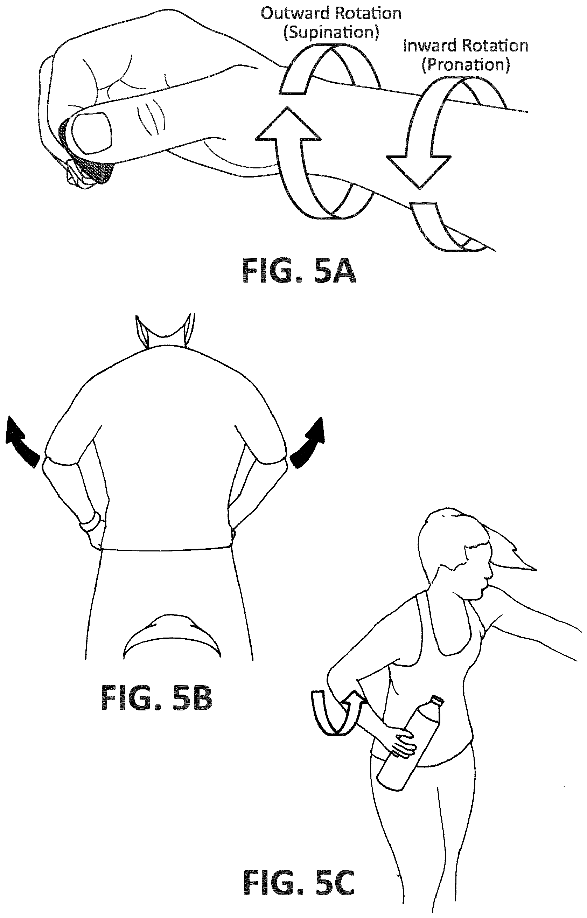

Figures 5A-5C depict other movements that can trigger the sensor 5, but which are more complex movements which can be performed holding objects. For example, figure 5A shows outward and inwards rotation of the forearm, which could be performed holding a cup. Figure 5B represents abduction and adduction with a hand in a fixed position, for example when riding a bike. Figure 5C presents elbow movement, while holding a water bottle.

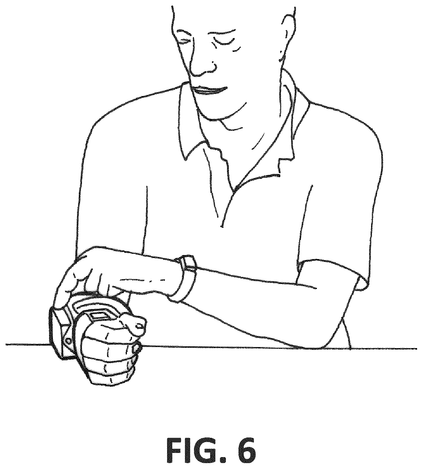

Finally, figure 6 presents the triggering of the stimulator 4 by direct tapping on it. The tapping can be done by the user him/herself with the arm that is not affected by the movement problem, or by a third person. The triggering of the stimulator 4 means that the embedded sensor 5 sends measurements to the control means 6 that they identify as an intention of the user to perform an action.

As previously explained, the sensor 5 continuously measures the presence or absence of the movement of the stimulator 4. The sensor sends its measurements to the control means 6, which determine, based on values of the sensor measurements, if there is an intention to perform an action. An intention to perform an action is inferred when the detected measurements are over a predetermined threshold, therefore only when a sharp movement is detected, which must be preceded and followed by a rest period. This configuration prevents false positives, which could occur with nonintentional movements that could activate the device 1.

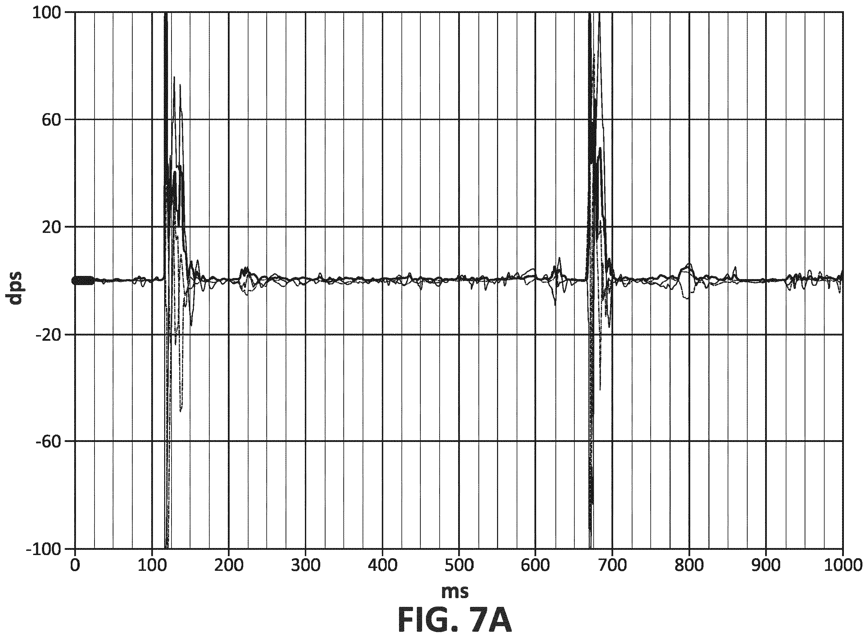

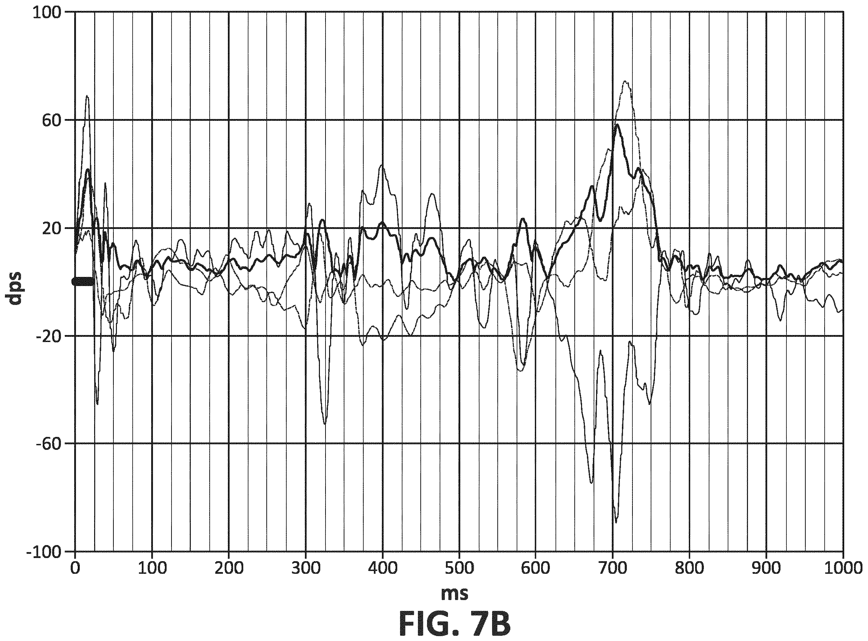

Figure 7A shows a graph of a movement (X axis represents time in milliseconds and axis Y represents dps (degrees per seconds)) which will be detected, which presents a sudden variation preceded and followed by a clear rest. On the contrary, figure 7B shows a graph (X axis also represents time in milliseconds and axis also Y represents dps (degrees per seconds)) of a movement which would not generate the trigger because there is no pre/post-motion rest period.

Figure 8 shows a detailed schematic representation of the electronics of an exemplary stimulator 4 of device 1. As shown in figure 8, the electronics comprise a touch circuit 8, a processing unit 28 and an output module 29. The touch circuit 8 comprises, in turn, touch buttons 9, as an interface between the user and the device 1, light indicators 10 and a sound unit 11, which facilitate the communication between the device 1 and the user.

The processing unit 28 of the exemplary electronics of the stimulator 4 further comprise, as presented in figure 8, control means 6, which could be, for example, a microcontroller, an ON/OFF controller 12, to activate or deactivate the device 1, a Bluetooth 4.0 unit 13 and/or a Bluetooth 5.0 unit 14, which facilitate the communication of the device 1 with external equipment. Optionally, a USB port 15, for extracting data from the device 1. Also, a temperature sensor 17, which can provide more data of interest of the state of the user. Moreover, to power the device 1, the processing unit 28 comprises a battery 18, a battery management unit 19, a +3v3 power port 20 and a +5V power 22 port, all of them associated with an external charger port 23 and a flash memory 21. The battery 18 charges through the USB port 15.

The exemplary electronics of the stimulator 4 also comprise a pulse generator 16, which is the one in charge of generating the electrical signals that, by means of the electrode 2, will activate the muscles of the user. The pulse generator 16 is connected to the output module 29 and, in particular, it is connected to anode and cathode selection modules 24 and 25, connected to a control unit 26 and to electrode connectors 27.

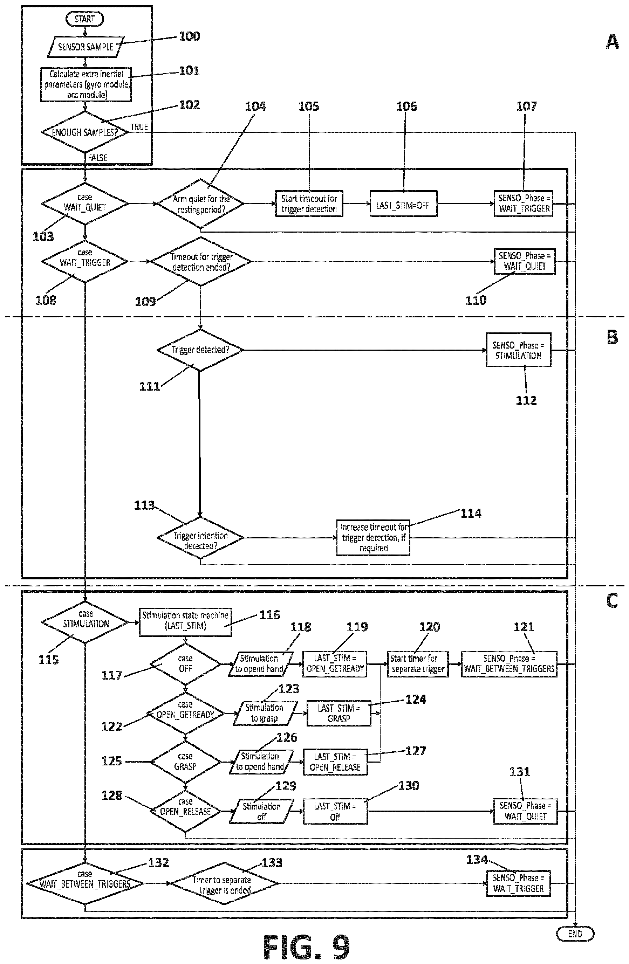

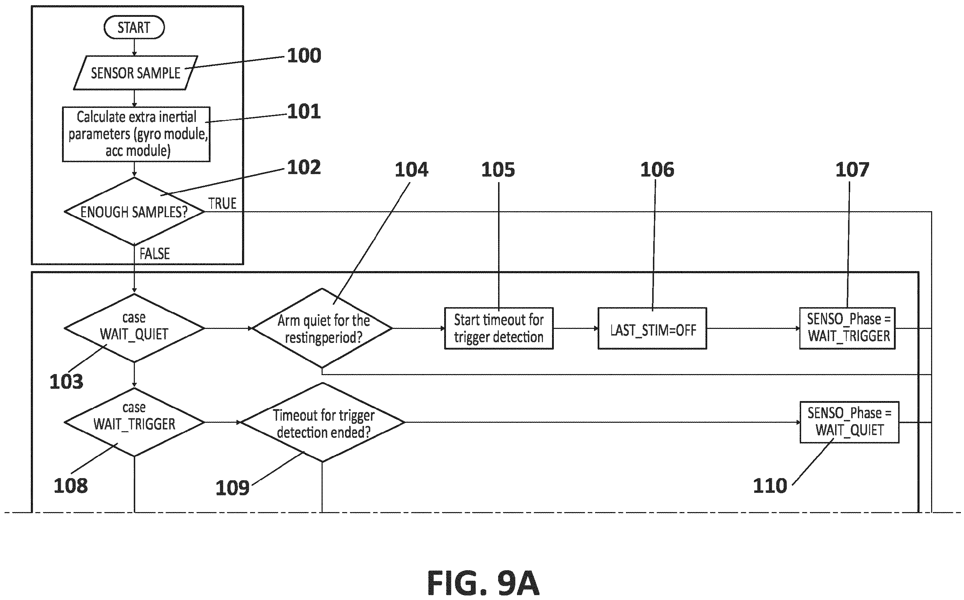

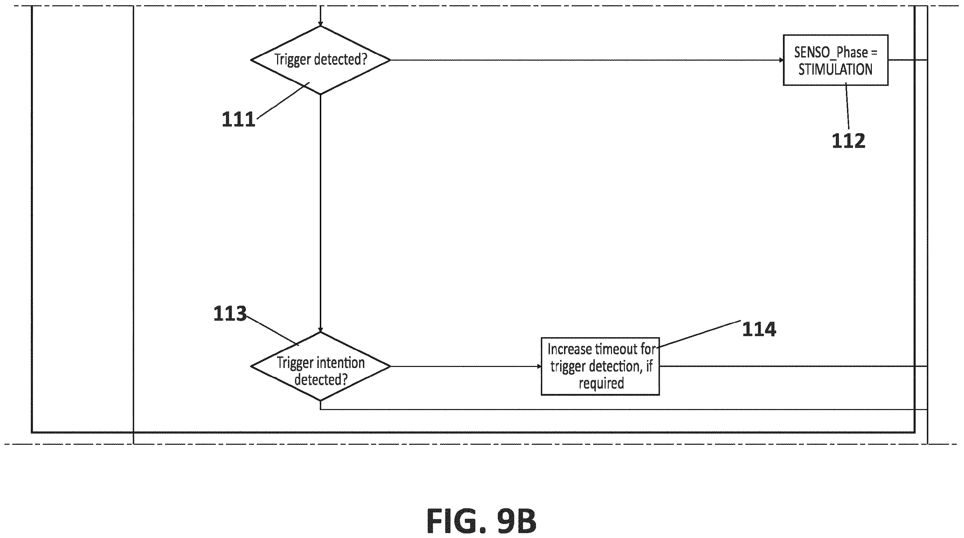

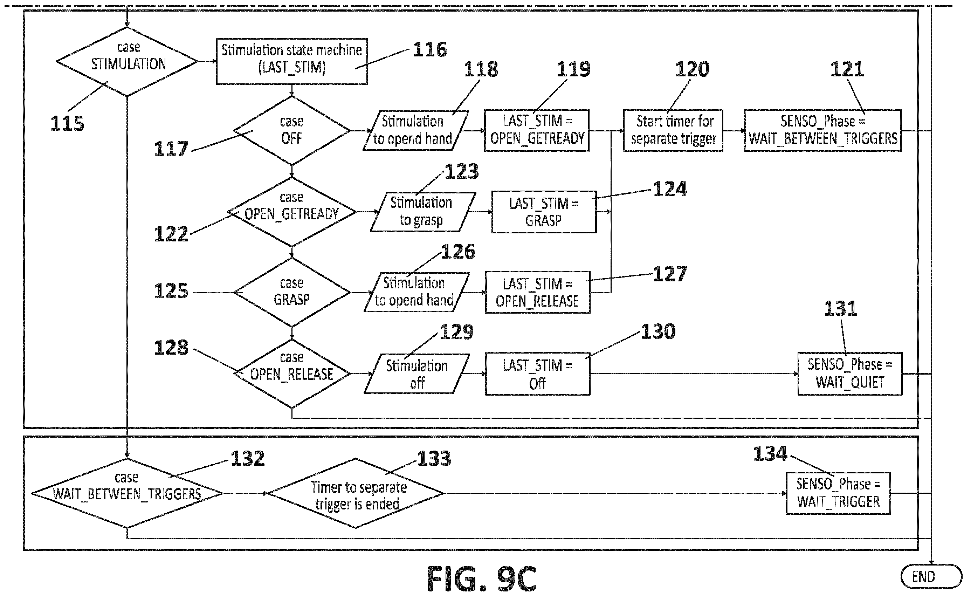

Figure 9 shows an exemplary embodiment for a method for rehabilitation of hand function by functional electrical stimulation according to this disclosure. The method can be performed by the device 1 as disclosed above. The sensor 5 of the device 1 continuously captures measurements of the movement of the stimulator 4 along time and provides sensor measurements, as shown for example in figures 7A and 7B. These measurements, which include the data obtained by a 3-axis gyroscope and a 3-axis accelerometer, are provided to the control means 6 (i.e., microprocessor), which processes the sensor measurements and from said measurements determines an intention of the user to perform an action with his/her hand or arm on which the electrode 2 is disposed. The control means 6 processes the sensor measurements meaning that it obtains the modulus of the 3-axis of the gyroscope and the derivate of the modulus of the axis of the accelerometer.

In this embodiment shown in figure 9, a sensor 5 sample 100 is obtained. Then, the control means 6 calculate the parameters previously explained (gyro module, acc module) 101 and if there are enough samples 102, the method proceeds to the next steps.

In a first case (WAIT_QUIET) 103 the control means 6 wait for a resting period. If during said resting period no movement has been detected 104, the control means 6 start a timeout for trigger detection 105. Then 107, the device 1 goes to the second case (WAIT_TRIGGER) 108.

In this second case (WAIT_TRIGGER) 108, if the timeout for trigger detection ends 109, the device 1 goes back 110 to the first case (WAIT_QUIET) 103.

In this second case (WAIT_TRIGGER) 108 if a trigger (a sharp movement) is detected 111, a stimulation step (STIMULATION) is activated 112, which is described below. In this case, the conditions to detect a trigger are the following:
1. Signal quiet before movement (during a first rest period):
   - average of gyroscope module below a first value of the measured parameters,
2. Arm movement (during a movement period):
   - max value of gyroscope above a second value of the measured parameters, and change in accelerometer
3. Signal quiet after movement (during a second rest period):
   - average of gyroscope module below a third value of the measured parameters,

In this second case (WAIT_TRIGGER) 108 if an intention trigger is detected 113, the stimulation step (STIMULATION) is activated, and the timeout for trigger detection is increased, if required 114.

The previously mentioned STIMULATION 115 step goes as follows:
- if STIMULATION was off 117, stimulation to open hand 118 is applied and the device 1 goes to an OPEN_GET READY state 119,
- If the state is OPEN_GETREADY 122, stimulation to grasp is activated 123 and the device 1 goes to a GRASP state 124,
- If the state is GRASP 125, stimulation to open hand is applied 126 and the device 1 goes to OPEN_RELEASE state 127.

All the previous steps start a timer to separate triggers 120, after which, the device 1 goes to a state of (WAIT_BETWEEN_TRIGGERS) 121.
- If the state is OPEN_RELEASE 128, stimulation is off 129, the device 1 goes to the first state (WAIT QUIET)131.

In the case (WAIT_BETWEEN TRIGGERS) 132, if the timer to separate triggers is ended 133, it goes to WAIT_TRIGGER 134.

In this text, the term "comprises" and its derivations (such as "comprising", etc.) should not be understood in an excluding sense, that is, these terms should not be interpreted as excluding the possibility that what is described and defined may include further elements, steps, etc.

The invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

The numerical signs and corresponding components indicated in Fig. 1 to 9 are further listed below:
Device 1
Electrode 2
Electrode pads 3
Stimulator 4
Embedded sensor 5
Control means 6
Textile layer 7
Touch circuit 8
Touch buttons 9
Light indicators 10
Sound unit 11
ON/OFF controller 12
Bluetooth 4.0 unit 13
Bluetooth 3.0 unit 14
USB 15
Pulse generator 16
Temperature sensor 17
Battery 18
Battery management unit 19
+3v3 Power 20
Flah memory 21
+5V Power 22
External charger 23
Anodes switches 24
Cathodes switches 25
Switches control 26
Electrode connectors 27
Processing unit 28
Output module 29

## Claims

1. A device (1) for functional electrical stimulation intended to be positioned on an upper limb of a user for stimulation of the user's upper limb, the device (1) comprising:
- an electrode (2) configured to be disposed on an upper limb of the user, the electrode (2) having a plurality of electrode pads (3) adapted to generate stimulating electrical signals on the arm of the user, and
- a stimulator (4) comprising:
- an embedded sensor (5) configured to, in use of the device, continuously measure movement of the stimulator (4), and to emit sensor measurements indicative thereof, and
- control means (6) connected to the embedded sensor (5) and configured to process the sensor measurements and from said measurements determine an intention of the user to perform an action with his/her upper limb on which the electrode (2) is disposed, and to control the activation and/or deactivation of at least one of the plurality of electrode pads (3) based on the sensor measurements.

2. The device (1) of claims 1, wherein the movement of the stimulator (4) associated with an intention of the user to perform an action with his/her upper limb is a sharp movement of the stimulator (4) preceded by a first rest period and followed by a second rest period.

3. The device (1) of any of the preceding claims wherein the movement of the stimulator (4) is generated by at least one of:
- impaired arm flexion, extension, abduction, adduction, medial rotation, lateral rotation, horizontal flexion/adduction, flexion or extension with or without hand in a fixed position,
- impaired forearm flexion, extension, pronation, semipronation, supination or prosupinantion, with or without hand in a fixed position,
- tapping on the stimulator (4).

4. The device (1) of any of the preceding claims, wherein the embedded sensor (5) comprises an IMU (inertial measurement unit).

5. The device of claim 4, wherein the IMU is a three axis IMU or a six axis IMU.

6. The device (1) of any of the preceding claims, wherein the embedded sensor (5) comprises an EMG sensor (electromyography).

7. A method for rehabilitation of upper limb function by functional electrical stimulation, the method comprising the steps of:
- providing a stimulator (4) having an embedded sensor (5),
- measuring by the sensor (5) movement of the stimulator (4) and emitting sensor measurements indicative thereof,
- providing the sensor measurements to control means (6),
- determining, with the control means (6), an intention of the user to perform an action with his/her impaired upper limb, wherein said intention to perform the action is determined based on the sensor measurements,
- if an intention to perform an action is determined, controlling, with the control means (6), the activation and/or deactivation of at least one of a plurality of electrode pads (4) comprised in an electrode (3) intended to be positioned on an upper limb or a user.

8. The method of claim 7, wherein the stimulator (4) is attached to a user's arm.

9. The method of any of claims 7-8, wherein the movement is generated in one of the following ways:
- impaired arm flexion, extension, abduction, adduction, medial rotation, lateral rotation, horizontal flexion/adduction, flexion or extension, with or without hand in a fixed position,
- impaired forearm flexion, extension, pronation, semipronation, supination or prosupinantion, with or without hand in a fixed position,
- tapping on the stimulator (4).

10. The method of any of claims 7-9, wherein the movement of the stimulator (4) associated with an intention of the user to perform an action with his/her upper limb is a sharp movement of the stimulator (4) preceded by a first rest period and followed by a second rest period.

11. The method of claim 10, wherein a waiting period following the second rest period or preceding the first rest period is defined, aimed at turning the device (1) to an active mode, and wherein during said waiting period the control means (6) do not send any signal to the electrode (2) to activate or deactivate the electrode pads (3).

12. The method of any of claims 7-11, wherein a movement associated with an intention of the user to perform an action with his/her upper limb is detected when the control means (6) identify a measurement from the sensor (5) with a value above a value of the measurements preceded by a first rest period and followed by a second rest period.

13. The method of claim 12, wherein the control means (28) identify an intention of the user to perform and action if the following conditions are met:
- during a first rest period the sensor measurements are below a first value of the measurements,
- during a movement period following the first rest period, the sensor measurements are above a second value of the measurements which is at least twice the first value,
- during a second rest period following the movement period the sensor measurements are below the first value of the measurements.

14. The method of claim 13, wherein the first value is between 0 and 12 dps (degrees per second), preferably between 0 and 6 dps and more preferably between 0 and 3 dps and the second value is above 15dps, provided that the second value is at least twice the first value.

15. The method of any of claims 13-14 wherein the first rest period is between 100 and 250ms, the movement period is between 250 and 1000ms and the second rest period is between 150 and 750ms.
